(19) Europäisches Patentamt
European Patent Office
Office européen des brevets

(11) **EP 4 043 027 A1**

(12) **EUROPEAN PATENT APPLICATION**
published in accordance with Art. 153(4) EPC

(43) Date of publication:
**17.08.2022 Bulletin 2022/33**

(21) Application number: **19947626.8**

(22) Date of filing: **31.12.2019**

(51) International Patent Classification (IPC):
**A61K 38/21** $^{(2006.01)}$ **A61K 39/00** $^{(2006.01)}$
**C07K 17/02** $^{(2006.01)}$ **A61P 35/00** $^{(2006.01)}$

(52) Cooperative Patent Classification (CPC):
**A61K 38/21; A61K 39/00; A61K 39/395;
A61K 45/06; A61P 35/00; C07K 17/02**

(86) International application number:
**PCT/CN2019/130747**

(87) International publication number:
**WO 2021/062960 (08.04.2021 Gazette 2021/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.09.2019 CN 201910943897**

(71) Applicant: **Shanghai Institute Of Biological
Products Co., Ltd
Shanghai 200052 (CN)**

(72) Inventors:
• **YANG, Yufeng**
**Shanghai 200051 (CN)**
• **HE, Cheng**
**Shanghai 200051 (CN)**
• **LOU, Jueren**
**Shanghai (CN)**

(74) Representative: **Rondano, Davide
Società Italiana Brevetti S.p.A.
Via Carducci 8
20123 Milano (IT)**

(54) **APPLICATION OF PEG INTERFERON AND PROTO-ONCOGENE PRODUCT TARGETING INHIBITOR IN SYNERGISTIC TREATMENT OF RENAL CARCINOMA**

(57) An application of PEG interferon and a proto-oncogene product targeting inhibitor in synergistic treatment of renal carcinoma. Specifically, provided is use of a combination of active components, the combination of active components comprising the PEG interferon and the proto-oncogene product targeted inhibitor, and the combination being used for preparing a pharmaceutical composition for synergistic treatment of renal carcinoma. The combination of active components can effectively and synergistically inhibit renal carcinoma, and can significantly reduce toxic and side effects, and thus can be widely applied to tumor targeted treatment.

EP 4 043 027 A1

**Description**

**Technical field**

The invention relates to the technical field of antitumor drugs, and in particular to the application and related drugs of PEG-interferons and proto-oncogene product targeting inhibitors in synergistic treatment of renal carcinoma.

**Background**

Tumor is a kind of systemic disease with multi-factor participation and multi-step development. Tumors have biological characteristics of high complexity, variability and diversity. Abnormalities of cancer-related genes can cause instability of tumor genome, which in turn leads to abnormal biological behaviors of tumor cells, such as evasion of apoptosis, the potential for unlimited replication, angiogenesis, invasion and metastasis, and immune escape.

At present, surgery, radiotherapy and chemotherapy are the main methods for the treatment of malignant tumors. Surgery and radiotherapy are local treatment methods, while drug treatment is a method with systemic effect, which takes into account the characteristics of spread and metastasis of malignant tumors.

Traditional tumor treatment methods, including surgery, radiotherapy and chemotherapy, have shown good efficacy, but also many disadvantages. In recent years, tumor immunotherapy has significantly improved the quality of life for patients compared with traditional tumor treatment.

However, the ineffective clinical treatment and the frequent emergence of drug resistance have prompted the need to develop more effective treatment regimens. In addition, some anti-tumor drugs have serious toxic and side effects, causing patients to give up the treatment.

Therefore, there is an urgent need in the art to develop new effective treatment methods and drugs for treating tumors with low toxic and side effects.

**Summary of the invention**

The purpose of the present invention is to provide an effective treatment method and medicine thereof for treating renal carcinoma with low toxic and side effects. Specifically, the present invention provides the use of a PEGylated (PEG) interferon and a proto-oncogene product targeting inhibitor in synergistic inhibition of renal carcinoma.

In the first aspect of the present invention, it provides the use of a combination of active ingredients, which comprises a first active ingredient of PEG-interferon and a second active ingredient of proto-oncogene product targeting inhibitor, and the combination is used in preparing a pharmaceutical composition or a kit for synergistic treatment of renal carcinoma.

In another preferred embodiment, the proto-oncogene product targeting inhibitor is an antibody.

In another preferred embodiment, the PEG-interferon is a PEG-interferon α1b.

In another preferred embodiment, the PEG-interferon comprises a recombinant human interferon alb modified with PEG of 10,000-100,000 molecular weight, preferably a recombinant human interferon alb modified with PEG of 10,000-50,000 molecular weight.

In another preferred embodiment, a mass ratio of the PEG-interferon to the proto-oncogene product targeting inhibitor is 1:100 to 500:1, preferably 1:20 to 200:1, and more preferably 1:10 to 100:1 or 1:5 to 50:1.

In another preferred embodiment, a ratio (IU/mg) of the PEG-interferon to the proto-oncogene product targeting inhibitor is 1,000 IU/mg to 100 million IU/mg, preferably 10,000 IU/mg to 10 million IU/mg, and more preferably 100,000 IU/mg to 5 million IU/mg or 200,000 IU/mg to 3 million IU/mg.

In another preferred embodiment, the pharmaceutical composition or kit is used in treatment of a mammal or is administered to a mammal, preferably rodent (e.g. mice, rats) or human.

In another preferred embodiment, the proto-oncogene product targeting inhibitor is selected from the group consisting of: an EGFR inhibitor, and a combination thereof.

In another preferred embodiment, the proto-oncogene product targeting inhibitor comprises Avastin.

In the second aspect of the present invention, it provides a pharmaceutical composition, which comprises a PEG-interferon, a proto-oncogene product targeting inhibitor and a pharmaceutically acceptable carrier.

In another preferred embodiment, the pharmaceutical composition further comprises an additional therapeutic drug (e.g. an antitumor agent).

In another preferred embodiment, the pharmaceutical composition further comprises a chemotherapeutic agent, a checkpoint inhibitor, CAR-T cells, and combinations thereof.

In another preferred embodiment, the chemotherapeutic agent comprises cisplatin or paclitaxel.

In the third aspect of the present invention, it provides a combination of active ingredients, which consists of a PEG-interferon and a proto-oncogene product targeting inhibitor.

In another preferred embodiment, the proto-oncogene product targeting inhibitor comprises an EGFR inhibitor.

In the fourth aspect of the present invention, it provides a kit comprising:

(a) a first preparation comprising a PEG-interferon and a pharmaceutically acceptable carrier;
(b) a second preparation comprising a proto-oncogene product targeting inhibitor and a pharmaceutically acceptable carrier; and
(c) an instruction describing a method of combining the PEG-interferon with the proto-oncogene product targeting inhibitor for tumor treatment.

In another preferred embodiment, the first preparation and the second preparation are separate from one another.

In another preferred embodiment, the first preparation and the second preparation are lyophilized preparation or liquid preparation.

In another preferred embodiment, the first preparation and the second preparation are injection.

In another preferred embodiment, the first preparation is administered before, during or after the administration of the second preparation.

In another preferred embodiment, the proto-oncogene product targeting inhibitor is selected from the group consisting of: an EGFR inhibitor, and combinations thereof.

In another preferred embodiment, the proto-oncogene product targeting inhibitor comprises Avastin.

In the fifth aspect of the present invention, it provides a non-therapeutic method *in vitro* for synergistic inhibition on the growth of tumor cells, which comprises the following steps: adding the combination of active ingredients according to the third aspect of the present invention and/or the pharmaceutical composition according to the second aspect of the present invention to a culture system for tumor cells, thereby synergistically inhibiting the growth of tumor cells.

In another preferred embodiment, the tumor cells include renal carcinoma cells and liver carcinoma cells.

In another preferred embodiment, the method comprises: co-culturing the tumor cells with the PEG-interferon and the proto-oncogene product targeting inhibitor.

In another preferred embodiment, the tumor cells comprise tumor cells in logarithmic growth phase.

In another preferred embodiment, the number of tumor cells is $10^3$-$10^8$ cells/ml.

In another preferred embodiment, the culture time is 0.1-120 hours, and preferably 1-96 hours.

In another preferred embodiment, a final concentration of the PEG-interferon in the mixed medium is 100-1,000 $\mu$g/ml, preferably 200-500 $\mu$g/ml, and more preferably about 300 $\mu$g/ml.

In another preferred embodiment, a final concentration of the proto-oncogene product targeting inhibitor in the mixed medium is 0.5-50 $\mu$g/ml, preferably 1-25 $\mu$g/ml, and more preferably 2-15 $\mu$g/ml, for example, about 3 $\mu$g/ml.

In another preferred embodiment, the proto-oncogene product targeting inhibitor is selected from the group consisting of: an EGFR inhibitor, and combinations thereof.

In another preferred embodiment, the proto-oncogene product targeting inhibitor comprises Avastin.

In the sixth aspect of the present invention, it provides a method for treating renal carcinoma, which comprises the following steps: administering the pharmaceutical composition according to the second aspect of the present invention and/or the combination of active ingredients according to the third aspect of the present invention to a subject (e.g., a human) in need, thereby treating tumor cells.

In the seventh aspect of the present invention, it provides a method for reducing toxic and side effects of a proto-oncogene product targeting inhibitor, which comprises the following steps: administering a PEG-interferon to a subject (e.g., a human, especially a patient with renal carcinoma) in need, wherein the PEG-interferon is administered to the subject before, during and/or after the administration of a proto-oncogene product targeting inhibitor.

In another preferred embodiment, the toxic and side effects are selected from the group consisting of: weight loss, gastrointestinal reactions (such as nausea, vomiting and loss of appetite), fatigue, rash and erythema.

In another preferred embodiment, the proto-oncogene product targeting inhibitor is selected from the group consisting of: an EGFR inhibitor, and combinations thereof.

In another preferred embodiment, the proto-oncogene product targeting inhibitor comprises Avastin.

It should be understood that within the scope of the present invention, the above-mentioned technical features of the present invention and the technical features specifically described in the following (such as the embodiments) can be combined with each other to form a new or preferred technical solution, which are not redundantly repeated one by one due to space limitation.

**Description of the drawings**

Figure 1 is a graph showing the therapeutic effects of using PEGylated (PEG) interferon alb alone or in combination with Avastin on subcutaneous transplanted tumors of human renal carcinoma Caki-1 in nude mice.

Figure 2 shows the effects of using PEGylated (PEG) interferon alb alone or in combination with Avastin on the body weights of nude mice with human renal carcinoma Caki-1.

Figure 3 is a photograph of tumors showing the therapeutic effects of PEGylated (PEG) interferon alb alone or in combination with Avastin on subcutaneous transplanted tumors of human renal carcinoma Caki-1 in nude mice.

Figure 4 shows the therapeutic effects of using PEGylated (PEG) interferon or Avastin alone or in combination on subcutaneous transplanted tumors of human renal carcinoma 786-O in nude mice.

Figure 5 shows the therapeutic effects of using PEGylated (PEG) interferon or Avastin alone or in combination on the body weights of 786-O tumor-bearing nude mice.

Figure 6 shows the therapeutic effects of using PEGylated (PEG) interferon or Avastin alone or in combination on subcutaneous transplanted tumors of human renal carcinoma Caki-1 in nude mice. Above: D21; below: D42.

Figure 7 shows the therapeutic effects of using PEGylated (PEG) interferon or Avastin alone or in combination on subcutaneous transplanted tumors of human liver cancer Huh-7 in nude mice.

Figure 8 shows the therapeutic effects of using PEGylated (PEG) interferon or Avastin alone or in combination on the body weights of Huh-7 tumor-bearing nude mice.

Figure 9 shows the therapeutic effects of using PEGylated (PEG) interferon or Avastin alone or in combination on subcutaneous transplanted tumors of human liver cancer Huh-7 in nude mice.

## Detailed Description

After extensive and intensive research and massive screening, the inventors have unexpectedly discovered for the first time that a PEGylated (PEG) interferon and a proto-oncogene product targeting inhibitor have a satisfactory synergistic effect in the treatment of tumors (especially renal carcinoma). The combined use of the PEGylated (PEG) interferon and the proto-oncogene product targeting inhibitor can effectively inhibit tumor growth, and its effect is far better than the additive effect of both. Besides, side effects which occur when using interferons (especially those caused by long-term use of interferon drugs at high doses) can be significantly reduced by the combined use. On this basis, the inventors have completed the present invention.

## Terms

As used herein, the term "a first active ingredient" refers to a PEGylated (PEG) interferon. It should be understood that the term comprises derivatives with the same function as PEGylated (PEG) interferons.

As used herein, the term "a second active ingredient" refers to a proto-oncogene product targeting inhibitor, especially a small molecule targeting inhibitor and an antibody-based targeting inhibitor. Preferably, the proto-oncogene product is a protein, such as EGFR protein, and the like.

As used herein, the terms "a combination of active ingredients of the invention", "a combination of substances of the invention" and "a combination of pharmaceutical ingredients of the invention" are used interchangeably, referring to a combination of the first active ingredient and the second active ingredient described above.

As used herein, the term "a pharmaceutical composition of the invention" refers to a composition which comprises a first active ingredient (or a first preparation comprising the first active ingredient) and a second active ingredient (or a second preparation comprising the second active ingredient), wherein the first preparation and the second preparation may be the same or different preparations. Furthermore, the first preparation and the second preparation may be the one single identical preparation or independent two preparations.

## Ordinary interferon and PEGylated (PEG) interferons

As an important member of tumor immunotherapy, ordinary interferons (IFN) therapy has been used in tumor treatment for many years. A large number of studies have shown that ordinary interferons have well performance in the inhibition of the growth of a variety of tumors.

Ordinary interferon $\alpha$ was approved as the first biological agent for the treatment of malignant tumors in 1986. Currently, it has a wide range of indications, including hairy cell leukemia, chronic leukemia, non-Hodgkin's lymphoma, myeloma, bladder cancer, ovarian cancer, advanced metastatic renal cancer and pancreatic malignant endocrine tumors, melanoma, laryngeal papilloma, basal cell carcinoma and Kaposi sarcoma, etc.

Ordinary interferon alb (rhIFNalb) is the first genetically engineered protein drug in China that was cloned and developed from the leukocytes of the umbilical cord blood of healthy Chinese people in the 1980s.

rhIFNalb consists of 166 amino acids with a relative molecular weight of 19.382 kDa, and it has broad-spectrum antiviral, antitumor and immunomodulatory activities. Depending on the results of clinical researches conducted in the 1990s and the effects of clinical research and application in the past few decades, it has been proved to have a certain therapeutic effect on certain tumors. However, treatment with interferons alone requires the use of high doses and causes serious side effects and poor patient compliance.

Amino acid sequences and homology analysis of interferon α isoforms HuIFNα-1b(166aa) ACCESSION: AAL35223 (SEQ ID No.: 1)

```
  1 CDLPETHSLD   NRRTLMLLAQ   MSRISPSSCL   MDRHDFGFPQ   EEFDGNQFQK
 51 APAISVLHEL   IQQIFNLFTT   KDSSAAWDED   LLDKFCTELY   QQLNDLEACV
101 MQEERVGETP   LMNADSILAV   KKYFRRITLY   LTEKKYSPCA   WEVVRAEIMR
151 SLSLSTNLQE   RLRRKE                                      166
```

HuIFNα-2a(165aa) ACCESSION: 1ITF_A (SEQ ID No.: 2)

```
  1 CDLPQTHSLG   SRRTLMLLAQ   MRKISLFSCL   KDRHDFGFPQ   EEFGNQFQKA

 51 ETIPVLHEMI   QQIFNLFSTK   DSSAAWDETL   LDKFYTELYQ   QLNDLEACVI
101 QGVGVTETPL   MKEDSILAVR   KYFQRITLYL   KEKKYSPCAW   EVVRAEIMRS
151 FSLSTNLQES   LRSKE 165
```

HuIFNα-2b(165aa) ACCESSION: 1RH2_A (SEQ ID No.: 3)

```
  1 CDLPQTHSLG   SRRTLMLLAQ   MRRISLFSCL   KDRHDFGFPQ   EEFGNQFQKA
 51 ETIPVLHEMI   QQIFNLFSTK   DSSAAWDETL   LDKFYTELYQ   QLNDLEACVI
101 QGVGVTETPL   MKEDSILAVR   KYFQRITLYL   KEKKYSPCAW   EVVRAEIMRS
151 FSLSTNLQES   LRSKE                                      165
```

Homology analysis: NCBI BLAST HuIFN-1b and HuIFN-2a: 81%

```
Query   1    CDLPETHSLDNRRTLMLLAQMSRISPSSCLMDRHDFGFPQEEFDGNQFQKAPAISVLHEL   60
             CDLP+THSL +RRTLMLLAQM +IS  SCL DRHDFGFPQEEF GNQFQKA  I VLHE+
Sbjct   1    CDLPQTHSLGSRRTLMLLAQMRKISLFSCLKDRHDFGFPQEEF-GNQFQKAETIPVLHEM   59

Query   61   IQQIFNLFTTKDSSAAWDEDLLDKFCTELYQQPNDLEACVMQEERVGETPLMNADSILAV   120
             IQQIFNLF+TKDSSAAWDE LLDKF TELYQQ NDLEACV+Q   V ETPLM DSILAV
Sbjct   60   IQQIFNLFSTKDSSAAWDETLLDKFYTELYQQLNDLEACVIQGVGVTETPLMKEDSILAV   119

Query   121  KKYFRRITLYLTEKKYSPCAWEVVRAEIVRSLSLSTNLQERLRRKE   166
             +KYF+RITLYL EKKYSPCAWEVVRAEI+RS SLSTNLQE LR KE
Sbjct   120  RKYFQRITLYLKEKKYSPCAWEVVRAEIMRSFSLSTNLQESLRSKE   165
```

HuIFN-1b and HuIFN-2b: 83%

```
Query    1    CDLPETHSLDNRRTLMLLAQMSRISPSSCLMDRHDFGFPQEEFDGNQFQKAPAISVLHEL    60
              CDLP+THSL +RRTLMLLAQM RIS  SCL DRHDFGFPQEEF GNQFQKA  I VLHE+
Sbjct    1    CDLPQTHSLGSRRTLMLLAQMRRISLFSCLKDRHDFGFPQEEF-GNQFQKAETIPVLHEM    59
Query   61    IQQIFNLFTTKDSSAAWDEDLLDKFCTELYQQPNDLEACVMQEERVGETPLMNADSILAV   120
              IQQIFNLF+TKDSSAAWDE LLDKF TELYQQ NDLEACV+Q   V ETPLMN DSILAV
Sbjct   60    IQQIFNLFSTKDSSAAWDETLLDKFYTELYQQLNDLEACVIQGVGVTETPLMNEDSILAV   119
Query  121    KKYFRRITLYLTEKKYSPCAWEVVRAEIVRSLSLSTNLQERLRRKE    166
              +KYF+RITLYL EKKYSPCAWEVVRAEI+RS SLSTNLQE LR KE
Sbjct  120    RKYFQRITLYLKEKKYSPCAWEVVRAEIMRSFSLSTNLQESLRSKE    165
```

Polyethylene Glycol (PEG) is a general term for ethylene glycol polymers with a linear or branched structure and an average molecular weight of more than 200 Da. It has the characteristics of high molecular weight, safety and non-toxicity, amphiphilicity, immune inertness and structural stability, etc. Chemical modification of a protein with polyethylene glycol can prolong the half-life of the protein *in vivo,* improve its stability, reduce immunogenicity and antigenicity thereof, and etc., without changing the amino acid composition of protein. It is a simple and effective method to improve the pharmacodynamic properties of protein drugs.

A preferred PEGylated (PEG) interferon alb is a recombinant human interferon alb modified with PEG of about 10,000-100,000 Daltons (e.g. about 20,000). A particularly preferred PEGylated (PEG) interferon alb is a recombinant human interferon alb modified with PEG having a molecular weight of about 20,000 Daltons, and is a new type of long-acting interferon alb drugs (see Chinese Patent ZL200410067652.3). It was observed *in vitro* that the PEGylated (PEG) interferon alb could inhibit the proliferation of tumor cells. In addition, it was observed *in vivo* that the PEGylated (PEG) interferon alb had obvious therapeutic effects on subcutaneous transplanted tumors in mice.

*In vitro* experiments showed that the PEGylated (PEG) interferon alb could inhibit the proliferation of tumor cells and exert its antitumor effects by activating JAK/STAT signal pathway. It could inhibit the growth of vascular endothelial cells and promote their apoptosis by inhibiting the expression of tumor angiogenesis factors.

The results of tumor-bearing mice experiments showed that subcutaneous injection of the PEGylated (PEG) interferon alb could dose-dependently inhibit the growth of subcutaneous transplanted tumors of human renal carcinoma Caki-1 in nude mice.

Moreover, when the PEGylated (PEG) interferon alb is used in combination with the monoclonal antibody agent Avastin, the tumor inhibition rate of Caki-1 can be significantly increased, indicating that PEGylated (PEG) interferon alb has a synergistic effect on the treatment of subcutaneous transplanted tumors of human renal carcinoma Caki-1 in nude mice with Avastin. Moreover, when the first active ingredient of PEGylated (PEG) interferon and the second active ingredient of proto-oncogene product targeting inhibitor are used in combination, it can not only effectively inhibit tumors, but also significantly reduce toxic and side effects, especially one or more adverse reactions selected from the group consisting of: influenza-like syndrome, gastrointestinal reactions (e.g., nausea, vomiting, loss of appetite), fatigue, rash (including non-specific rash with pruritus), erythema, etc., and weight loss.

The study of the present invention also shows that a PEGylated (PEG) interferon can inhibit the growth and/or migration of tumor cells when administered to the tumor cells.

**Proto-oncogene product targeting inhibitor**

As used herein, the terms "proto-oncogene product targeting inhibitor" and "protooncoprotein targeting inhibitor" are used interchangeably and refer to inhibitors that specifically target the encoding products (especially proteins) of the proto-oncogene. As used herein, the term "protooncoprotein" refers to a protein encoded by a proto-oncogene, especially a membrane protein. In some embodiments of the present invention, a protooncoprotein inhibitor may exert its functions by reducing the amount of a protooncoprotein, inhibiting or blocking the activation of a protooncoprotein, or inhibiting other molecules in a signaling pathway.

Non-limiting embodiments of a proto-oncogene product targeting inhibitor include, (but are not limited to,) EGFR-targeting inhibitors, Her2-targeting inhibitors, and combinations thereof.

As used herein, the term "protooncoprotein activity" refers to the ability of a protooncoprotein to promote the formation, growth, proliferation, migration and/or invasion of cancer cells.

As used herein, the term "protooncoprotein expression" refers to the formation of a protooncoprotein gene product as measured by any method known in the art, including (but not limited to): nucleic acid hybridization methods, Northern blotting, protooncoprotein site hybridization, polymerase chain reaction amplification, reporter gene expression, etc. The formation of protooncoprotein gene products can also be measured by any antibody-based technique, including immunohistochemistry, immunofluorescence, Western blotting, and ELISA methods.

In the present invention, targeting inhibitors include (but are not limited to): antibodies, small molecule drugs, nucleic acid drugs, CAR-T cells, and the like.

## EGFR and inhibitors thereof

Epidermal growth factor receptor (EGFR) is a transmembrane protein (NM201282) and a member of ErbB receptor family. Its ligands (EGF, TGF-$\alpha$, etc.) bind to the extracellular segment of EGFR to dimerize, which leads to the activation of intracellular tyrosine kinases and a series of cascade reaction in signal transduction, i.e. promoting cell proliferation, angiogenesis, metastasis and inhibition of apoptosis, and thereby leading to tumorigenesis. In a large number of studies, it is found that EGFR gene is overexpressed or abnormally activated in various tumor tissues, thereby enhancing the ability of tumor cells to proliferate, invade and metastasize. EGFR has become a clinically validated therapeutic target for many types of tumors.

In the development of target treatment for cancer, a successful strategy is using monoclonal antibodies (mAbs) to localize epitopes on the surface of tumor cells, so as to target then kill cancer cells. Therefore, antibodies or other targeting inhibitors may be used for targeting EGFR on the surface of tumor cells to kill or inhibit them.

A representative EGFR inhibitor is an anti-EGFR antibody such as Bevacizumab (Avastin), biosimilars or ADC thereof. The Bevacizumab is an anti-EGFR monoclonal antibody.

## RNAi, siRNA, shRNA

In the present invention, nucleic acid drugs targeting proto-oncogenes can also be used to target and inhibit the proto-oncogenes (e.g., EGFR).

Representative nucleic acid drugs include (but are not limited to): RNAi, siRNA, shRNA, and precursors or expression vectors thereof.

As used herein, the term "RNAi" (RNA interference) refers to a phenomenon of efficient and specific degradation of homologous mRNA, which is highly conserved in evolution and induced by double-stranded RNA (dsRNA). Since the use of RNAi technology can specifically knock out or turn off the expression of specific genes, this technology has been widely used in exploring gene functions and in the field of gene therapy for infectious diseases and malignant tumors.

As used herein, the term "siRNA" (small interfering RNA, siRNA) refers to a small RNA molecule (about 21-25 nucleotides) that can be processed by Dicer (an enzyme in the RNAase III family that is specific to double-stranded RNA) from its precursors (such as dsRNA, shRNA, etc.), and can also be synthesized by chemical methods or processed by other proteins. siRNA is a main member of siRISC, which stimulates its complementary target mRNA to be rapidly cleaved and degraded, resulting in the silencing of the target gene, thus becoming a key functional molecule in RNAi.

As used herein, the term "expression cassette" refers to an expression cassette comprising a coding sequence of an RNAi precursor of the present invention and a promoter and termination signal operably linked to the coding sequence, which generates the RNAi precursor of the present invention after transcription. The term "RNAi precursor" as used herein refers to an RNA molecule that can be processed in mammalian cells to produce siRNA. Specifically, the RNAi precursor is selectively processed by Dicer, Ago2 or other similar proteins to produce mature siRNA for RNAi.

As used herein, the term "shRNA" is an abbreviation for short hairpin RNA. shRNA consists of two short reverse complementary sequences, and the middle of the shRNA is separated by an apical loop sequence, forming a hairpin structure. The transcription of shRNA is regulated by an endogenous RNA polymerase III promoter, and the end of the shRNA sequence is connected with 5-6 T as a transcription terminator of the RNA polymerase III. One way to generate a "small interfering RNA" (siRNA) *in vivo* is to clone the siRNA sequence into a plasmid vector as part of a "short hairpin". When delivered into animals, the hairpin sequence is expressed to form a "double-stranded RNA" (shRNA) with an apical loop structure, which is recognized and processed by proteins such as Dicer and Ago2 in cells to produce a functional siRNA.

As used herein, the term "miRNA" (microRNA) is a class of non-coding single-stranded RNA molecules of about 20-24 nucleotides in length encoded by endogenous genes, which are involved in the expression regulation of a large number of genes in animals and plants. So far, more than 4,000 miRNA molecules have been found in animals, plants and viruses. Most miRNA genes exist in the genome in the form of single copy, multiple copies or gene clusters. Each miRNA can regulate multiple target genes, and several miRNAs can also participate in regulating the same gene together, forming a complex regulatory network. It is speculated that miRNAs regulate the expression of more than half of human genes. There are many forms of miRNA, and the most primitive one is a pri-miRNA. After the pri-miRNA is processed by Drosha, it becomes a pre-miRNA, i.e., a miRNA precursor, with a length of about 50-90 nucleotides. After the pre-miRNA is digested by Dicer enzyme, it becomes a mature miRNA with a length of about 20-24 nucleotides. The miRNA inhibits the expression of a target gene mainly by inhibiting translation and accelerating deadenylation of the mRNA, and the mechanism thereof is different from siRNA-mediated mRNA degradation.

## Expression vector

As used herein, the term "expression vector" refers to a vector capable of transferring a polynucleotide sequence of interest to a target cell. Such vectors can self-replicate or integrate into host cell chromosomes (host cells include, e.g., prokaryotic cells, yeasts, animal cells, plant cells, insect cells, animal individuals, and plant individuals, etc.), and may contain promoters at sites suitable for transcription of the polynucleotides of the present invention. Expression vectors may contain structural genes and promoters that regulate their expression. Moreover, various regulatory elements that function in the host cells may also be contained. It is well known in the art that the type of expression vectors for a living organism (e.g., an animal) and the type of regulatory elements used can vary depending on the type of host cells used.

A viral vector that can be used in the present invention is not particularly limited, and may be any viral vector that can transmit its genome based on its characteristics to introduce genetic materials into other cells for infection. The introduction of genetic materials into other cells can occur in a whole, living organism or in cell culture. Viral vectors comprise lentiviral vectors, adenoviral vectors, herpesvirus vectors, and poxvirus vectors. In the present invention, a preferred expression vector is a lentiviral vector.

## Pharmaceutical composition and administration method

In the present invention, it also provides a composition that can be used in synergistic treatment for renal carcinoma, which can be used to inhibit the growth and/or metastasis of the tumors.

The pharmaceutical composition of the present invention comprises: an effective amount of a PEGylated (PEG) interferon, an effective amount of a proto-oncogene product targeting inhibitor, and a pharmaceutically acceptable carrier.

In addition, the pharmaceutical composition of the present invention may also comprises optional chemotherapeutic agents for solid tumors. The chemotherapeutic agents include (but are not limited to) cisplatin, paclitaxel, doxorubicin and the like.

In general, the PEGylated (PEG) interferon, or the proto-oncogene product targeting inhibitor of the present invention can be formulated in a non-toxic, inert and pharmaceutically acceptable carrier medium, wherein the pH is usually about 5-8, preferably, the pH is about 6-8.

As used herein, "pharmaceutically acceptable carrier" refers to a carrier for administration of a therapeutic agent, comprising various excipients and diluents.

The term refers to pharmaceutical carriers which are not themselves essential active ingredients and are not unduly toxic after administration. Suitable carriers are well known to those skilled in the art. Pharmaceutically acceptable carriers in the composition may contain liquids such as water, saline, and buffers. In addition, auxiliary substances such as fillers, lubricants, glidants, wetting or emulsifying agents, pH buffering substances and the like may also be present in these carriers. The carriers may also contain cell transfection reagents.

As used herein, the term "effective amount" or "effective dose" refers to an amount that is functional or active in humans and/or animals and/or cells and is acceptable to humans and/or animals.

As used herein, a "pharmaceutically acceptable" ingredient is a substance that is suitable for use in humans and/or mammals without undue adverse side effects (such as toxicity, irritation, and allergy), i.e., a substance with a reasonable benefit/risk ratio. The term "pharmaceutically acceptable carrier" refers to a carrier for administration of a therapeutic agent, comprising various excipients and diluents. Such carriers include (but are not limited to) saline, buffers, dextrose, water, glycerol, polysorbates, ethanol, and combinations thereof. Usually, a pharmaceutical preparation should be matched to the administration method, and the pharmaceutical composition of the present invention can be prepared in the form of injection, for example, prepared by conventional methods with normal saline or an aqueous solution containing glucose and other adjuvants. The pharmaceutical composition is preferably manufactured under sterile conditions. The dosage of an active ingredient is a therapeutically effective amount. The pharmaceutical preparation of the present invention may also be made into a sustained-release preparation.

Furthermore, a composition of active ingredients of the present invention may also be used with other therapeutic agents (such as antineoplastic or immunomodulatory agents).

When using a pharmaceutical composition, a safe and effective amount of a combination of active ingredients (comprising the first active ingredient (or a formulation thereof) and/or the second active ingredient (or a formulation thereof)) is administered to the mammals.

It should be understood that the effective amount of the first active ingredient (or a formulation thereof) and/or the second active ingredient (or a formulation thereof) in the combination of active ingredients of the present invention may vary with the mode of administration and the severity of the tumor, etc. Selection of the preferred effective amount can be determined by those skilled in the art based on various factors (e.g., through clinical trials). Such factors include, but are not limited to: pharmacokinetic parameters such as bioavailability, metabolism, half-life, etc.; severity of the tumor, patient's weights, patient's immune status, route of administration, and the like.

Typically, for the first active ingredient, a safe and effective amount is usually at least about 10 ng/kg body weight, and in most cases is no more than about 50 mg/kg body weight, preferably the dose is about 50 ng/kg body weight - about 10 mg/kg body weight. Of course, the specific dose should also take into account the administration method, the patient's health and other factors, which are all within the skill of a skilled physician.

For the second active ingredient, a safe and effective amount is usually at least about 10 ng/kg body weight, and in most cases is no more than about 50 mg/kg body weight, preferably the dose is about 50 ng/kg body weight - about 10 mg/kg body weight. Of course, the specific dose should also take into account the administration method, the patient's health and other factors, which are all within the skill of a skilled physician.

The administration method of the pharmaceutical composition of the present invention is not particularly limited, and representative examples include (but are not limited to): intravenous injection, subcutaneous injection, intramuscular injection, and the like.

**Kit**

The present invention provides a kit comprising:

component (1): a preparation comprising a PEGylated (PEG) interferon;
component (2): a preparation comprising a proto-oncogene product targeting inhibitor; and
component (3): an instruction.

The preparation comprising a PEGylated (PEG) interferon includes (but is not limited to): lyophilized preparation, liquid preparation or intravenous injection.

The preparation comprising a proto-oncogene product targeting inhibitor includes (but is not limited to): lyophilized preparation, liquid preparation, tablets, capsules, suppositories, or intravenous injection.

In the present invention, a protein preparation is usually a lyophilized preparation or injection.

Typically, a kit contains one or more (e.g., at least two) units of dosage forms comprising a PEGylated (PEG) interferon and one or more (e.g., at least two) units of dosage forms comprising a proto-oncogene product targeting inhibitor; preferably each has 4-10 units of dosage forms.

As used herein, the term "unit of dosage form" refers to a composition prepared for convenience of administration in a dosage form required for a single administration, including but not limited to various solid agents (e.g., tablets), liquid agents, capsules, sustained-release agents.

The instruction of the present invention may comprise the following description: the use method of the kit is to use a unit of dosage form comprising a PEGylated (PEG) interferon and a unit of dosage form comprising a proto-oncogene product targeting inhibitor at the same time.

The kit of the present invention is prepared by the following steps: placing a preparation comprising a PEGylated (PEG) and a preparation comprising a proto-oncogene product targeting inhibitor together with the instruction, thereby forming the kit.

The preparation comprising a PEGylated (PEG) interferon preferably comprises a unit of dosage form comprising a PEGylated (PEG) interferon, and the preparation comprising a proto-oncogene product targeting inhibitor preferably comprises a unit of dosage form comprising a proto-oncogene product targeting inhibitor.

In the steps, preferably, at least one unit dosage form comprising the PEGylated (PEG) interferon and at least one unit dosage form comprising the proto-oncogene product targeting inhibitor, together with the instruction, are placed together to form the kit.

**Beneficial effects of the present invention**

1. A PEGylated (PEG) and a proto-oncogene product targeting inhibitor can act synergistically in anti-tumor therapy, thereby inhibiting tumor growth more significantly.
2. The combined use of a PEGylated (PEG) interferon and a proto-oncogene product targeting inhibitor can significantly reduce the dosage of the PEGylated (PEG) interferon and can show faster and stronger anti-tumor therapeutic effects. Therefore, it can significantly reduce the side effects caused by long-term and/or high-dose use of interferons.

The present invention will be further illustrated below with reference to the specific examples. It should be understood that these examples are only to illustrate the invention, not to limit the scope of the invention. The experimental methods with no specific conditions described in the following examples are generally performed under the conventional conditions e.g., the conditions described by Sambrook *et al.,* Molecular Cloning: Laboratory Guide (New York: Cold Spring Harbor Laboratory Press, 1989), or according to the manufacture's instructions. Unless indicated otherwise, all percentage and parts are calculated by weight.

**Reagents**

In each embodiment, the PEG-interferon refers to a PEGylated (PEG) interferon alb (Shanghai Institute of Biological Products Co., Ltd.), white lyophilized powder injection, 100 μg/bottle (0.5ml), 100,000 IU in total (i.e., 1,000 IU/μg).

Avastin (Bevacizumab injection) is a commercially available product.

**General method**

The use and welfare of laboratory animals were carried out in accordance with the regulations of the Association for the Assessment and Accreditation of Laboratory Animal Care International (AAALAC). The health status and death of the animals were monitored daily. Routine inspections included observing the effects of the test substances and drugs on animals' daily behaviors, such as behavioral activities, weight changes, and physical signs.

The indicators of tumor inhibition experiments in mice were to investigate the effect of drugs on tumor growth, and the specific indicators were T/C% or the rate of tumor growth inhibition TGI (%).

The diameter of the tumor was measured with a vernier caliper twice a week, and the formula for calculating the tumor volume (V) was: $V=1/2 \times a \times b^2$, wherein a and b represented the length and width, respectively.

$$T/C \ (\%) = (T\text{-}T_0)/(C\text{-}C_0) \times 100,$$

wherein, T and C were the tumor volumes at the end of the experiment; To and Co were the tumor volumes at the beginning of the experiment.

The rate of tumor growth inhibition (TGI) (%) =100-T/C (%).

When the tumor regressed, the rate of tumor growth inhibition (TGI) (%) was =100-$(T\text{-}T_0)/T_0 \times 100$

If a tumor was smaller than the initial volume, i.e., T<To or C<Co, it was defined as partial regression (PR) of the tumor; if a tumor disappeared completely, it was defined as complete regression (CR) of the tumor.

At the end of the experiment (e.g., D21), reaching the experimental endpoint, or the tumor volume reached 1,500 mm$^3$, the animals were sacrificed after $CO_2$ anesthesia, and then the tumors were dissected and photographed.

**Example 1**

**Antitumor experiment on renal carcinoma Caki-1 using PEGylated (PEG) interferon α1b alone or in combination with Avastin**

**1) Experimental animals and feeding thereof**

BALB/c nude mice, 4-5 weeks old, male, purchased from Shanghai Lingchang Biotechnology Co., Ltd. During the experiment, the mice were bred in the SPF grade animal room of the Shanghai Institute of Medicine, Chinese Academy of Sciences.

**2) Cells**

Human renal carcinoma Caki-1 cells were purchased from American Type Culture Collection. Cells were preserved and used by the Shanghai Institute of Medicine, Chinese Academy of Sciences.

**3) Test drugs**

PEGylated (PEG) interferon alb for injection (referred to as "PEGylated (PEG) interferon"): white lyophilized powder injection, 100 μg/bottle (0.5 ml), batch number S20170908; protected from light, sealed and stored at 2-8°C which was produced and provided by Shanghai Institute of Biological Products Co., Ltd.

Avastin (Bevacizumab injection): colorless and clear liquid, 100 mg (4 ml)/bottle; batch number H0182B03; protected from light, sealed and stored at 2-8°C, which was purchased from Roche Pharmaceuticals.

Each bottle of PEGylated (PEG) interferon alb was added with 120 μl of normal saline at a concentration of 1 mg/ml; prepared before use, and diluted with normal saline; Avastin was at a concentration of 25 mg/ml and was directly diluted with normal saline to the desired concentration.

**4) Construction of nude mice models with human renal carcinoma Caki-1**

Nude mice were subcutaneously inoculated with $8\times10^6$ human renal carcinoma Caki-1 cells. After the tumor grew to 100-150 mm$^3$, the animals were grouped according to tumor volumes.

**5) Tumor inhibition experiment process**

Mice were administered subcutaneously (SC) or intravenously (IV), twice a week (BIW); the administration volume was 10 mL/kg; the solvent group was administered with the same volume of "solvent" (normal saline). Specific dosage and administration regimen were shown in Table 1. The tumor volumes were measured twice a week, the mice were weighed, and the data were recorded.

**6) Results**

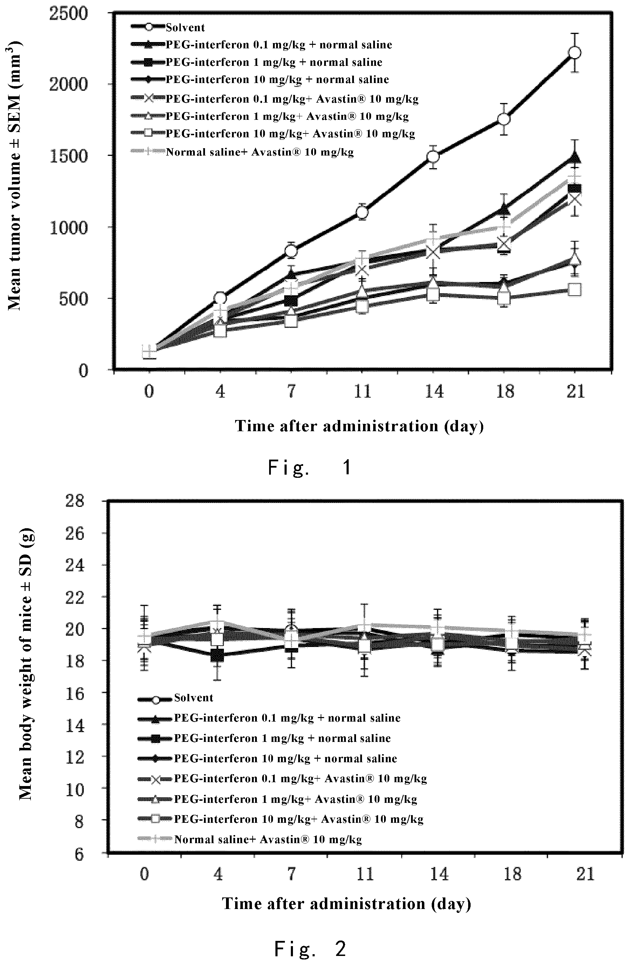

The results were shown in Table 1 and Figures 1-3.

Table 1. Therapeutic effects of using PEGylated (PEG) interferon $\alpha$1b alone or in combination with Avastin on subcutaneous transplanted tumors of human renal carcinoma Caki-1 in nude mice.

| Groups | Administration | Routes | Mean tumor volume (mm$^3$) | | Mean tumor % volume (mm$^3$) T/C | | | Tumor inhibition rate% | P value | Per group |
|---|---|---|---|---|---|---|---|---|---|---|
| | | | D0 | SEM | D21 | SEM | D21 | D21 | D21 | Number of animals |
| Solvent | D0,3,7,10,14,17 | SC/IV | 122.7 $\pm$ | 2.4 | 2215.3 $\pm$ | 135.8 | - | - | - | 12 |
| PEGylated (PEG) interferon 0.1 mg/kg | D0,3,7,10,14,17 | SC/IV | 120.5 $\pm$ | 3.8 | 1490.1 $\pm$ | 118.8 | 65 | 35 | 0.004 | 6 |
| PEGylated (PEG) interferon 1 mg/kg | D0,3,7,10,14,17 | SC/IV | 118.4 $\pm$ | 3.7 | 1252.6 $\pm$ | 143.9 | 54 | 46 | 0.000 | 6 |
| PEGylated (PEG) interferon 10 mg/kg | D0,3,7,10,14,17 | SC/IV | 119.0 $\pm$ | 5.4 | 753.5 $\pm$ | 86.8 | 30 | 70 | 0.000 | 6 |
| PEGylated (PEG) interferon 0.1 mg/kg+ Avastin 10 mg/kg | D0,3,7,10,14,17 | SC/IV | 122.4 $\pm$ | 3.2 | 1192.2 $\pm$ | 119.7 | 51 | 49 | 0.000 | 6 |
| PEGylated (PEG) interferon 1 mg/kg+ Avastin 10 mg/kg | D0,3,7,10,14,17 | SC/IV | 123.2 $\pm$ | 3.1 | 771.5 $\pm$ | 125.2 | 31 | 69 | * 0.000 | 6 |
| PEGylated (PEG) interferon 10 mg/kg+ Avastin 10 mg/kg | D0,3,7,10,14,17 | SC/IV | 122.2 $\pm$ | 3.2 | 554.7 $\pm$ | 32.1 | 21 | 79 | 0.000 | 6 |
| Avastin 10 mg/kg | D0,3,7,10,14,17 | SC/IV | 124.8 $\pm$ | 2.9 | 1348.1 $\pm$ | 64.5 | 58 | 42 | 0.001 | 6 |

D0: time of first administration; P value referred to the comparison with solvent; *$P < 0.05$, compared with PEGylated (PEG) 1 mg/kg or Avastin 10 mg/kg.

PEGylated (PEG) interferon $\alpha$1b (0.1, 1, 10 mg/kg, SC, twice a week, 6 times in total) dose-dependently inhibited the growth of subcutaneous transplanted tumors of human renal carcinoma Caki-1 in nude mice, and the tumor inhibition rates were 35%, 46% and 70%, respectively. The tumor inhibition rate of Avastin (10 mg/kg, IV, 2 times a week, 6 times in total) on Caki-1 subcutaneous transplanted tumors was 42%.

When PEGylated (PEG) interferon alb (0.1, 1, 10 mg/kg, SC, twice a week, 6 times in total) was used in combination with Avastin (10 mg/kg, IV, 2 times a week, 6 times in total), the tumor inhibition rate of Caki-1 was increased to 49%, 69% and 79%, respectively, indicating that PEGylated (PEG) interferon $\alpha$1b had a synergistic effect on subcutaneous transplanted tumors of human renal carcinoma Caki-1 in nude mice treated with Avastin, wherein PEGylated (PEG) interferon $\alpha$1b in medium dose of 1 mg/kg had a significant enhancement effect on Avastin ($P < 0.05$, compared with PEGylated (PEG) interferon $\alpha$1b or Avastin monotherapy), showing a synergistic effect.

In addition, tumor-bearing mice showed good tolerance to the drugs when they were used in combination, and no symptoms such as weight loss occurred. Especially as shown in Figure 2, the body weights of the mice in the combination group of PEGylated (PEG) interferon $\alpha$1b (1 mg/kg, SC, 2 times a week, 6 times in total) and Avastin (10 mg/kg, IV, 2 times a week, 6 times in total) were higher than the body weights of the mice in the Avastin alone group, which suggested that when PEG-interferons and Avastin were used in combination, it could not only effectively improve the therapeutic effects, but also significantly reduce the corresponding side effects.

The above experimental results showed that PEGylated (PEG) interferon $\alpha$1b combined with Avastin had a synergistic effect on subcutaneous transplanted tumors of human renal carcinoma Caki-1 in nude mice, and the mice showed good tolerance and fewer side effects, without symptoms like weight loss and so on.

Example 2

**Therapeutic effects of using PEGylated (PEG) interferon $\alpha$1b for injection alone or in combination with Avastin on subcutaneous transplanted tumors of human renal carcinoma 786-O in nude mice**

**1. Summary**

The therapeutic effects of using PEGylated (PEG) interferon $\alpha$1b for injection (referred to as "PEGylated (PEG) interferon") alone or in combination with Avastin on subcutaneous transplanted tumors of human renal carcinoma 786-O in nude mice were evaluated. PEGylated (PEG) interferon (0.1, 1, 10 mg/kg, SC, 2 times a week, 5 times in total); Avastin® (10 mg/kg, IV, 2 times a week, 5 times in total); or PEGylated (PEG) interferon (1 mg/kg, SC, 2 times a week, 5 times in total) and Avastin® (10 mg/kg, IV, 2 times a week, 5 times in total) in combination were used.

**2. Experimental purpose**

To evaluate the therapeutic effects of using PEGylated (PEG) interferon alone or in combination with Avastin on subcutaneous transplanted tumors of human renal carcinoma 786-O in nude mice.

**3. Test drugs**

**3.1 Drug Information**

PEGylated (PEG) interferon $\alpha$1b for injection (referred to as "PEGylated (PEG) interferon"): the same as that in Example 1.

Avastin (Bevacizumab injection): the same as that in Example 1.

**3.2 Suppliers**

The same as those Example 1.

**3.3 Drug formulation**

Each bottle of PEGylated (PEG) interferon was added with 120 $\mu$l of sterile water for injection at a concentration of 1 mg/ml, prepared before use, and diluted with normal saline; Avastin was at a concentration of 25 mg/ml and was directly diluted with normal saline to the desired concentration.

**4. Cells**

Human renal carcinoma 786-O cells were purchased from the Chinese Academy of Sciences Cell Bank, and the culture conditions were RPMI 1640 medium with 10% fetal bovine serum, penicillin and streptomycin, and cultured at 37°C in an incubator with air containing 5% $CO_2$. Cell passage was carried out 2-3 times a week, and when cells were in exponential growth phase, the cells were then digested with trypsin, collected, counted and seeded.

**5. Experimental animals**

NU/NU mice, 18-20 g, ♂, were purchased from Shanghai Branch of Beijing Weitong Lihua Laboratory Animal Technology Co., Ltd. Production license number: SCXK (Hu) 2017-0011; animal certificate number 20170011002819. Breeding environment: SPF grade.

**6. Experimental steps**

Nude mice were subcutaneously inoculated with $7\times10^6$ human renal carcinoma 786-O cells. After the tumor grew to 100-150 mm$^3$, the animals were grouped according to tumor volume (D0). Mice were administered subcutaneously (SC) or intravenously (IV), twice a week (BIW); the administration volume was 10 mL/kg; the solvent group was administered with the same volume of "solvent" (normal saline); specific dosage and administration regimen were shown in Table 2. The tumor volumes were measured twice a week, the mice were weighed, and the data were recorded.

**7. Statistical analysis**

Two-tailed Student's t-test was used to compare tumor volumes between the two groups, and $P<0.05$ was defined as a statistically significant difference.

**8. Results**

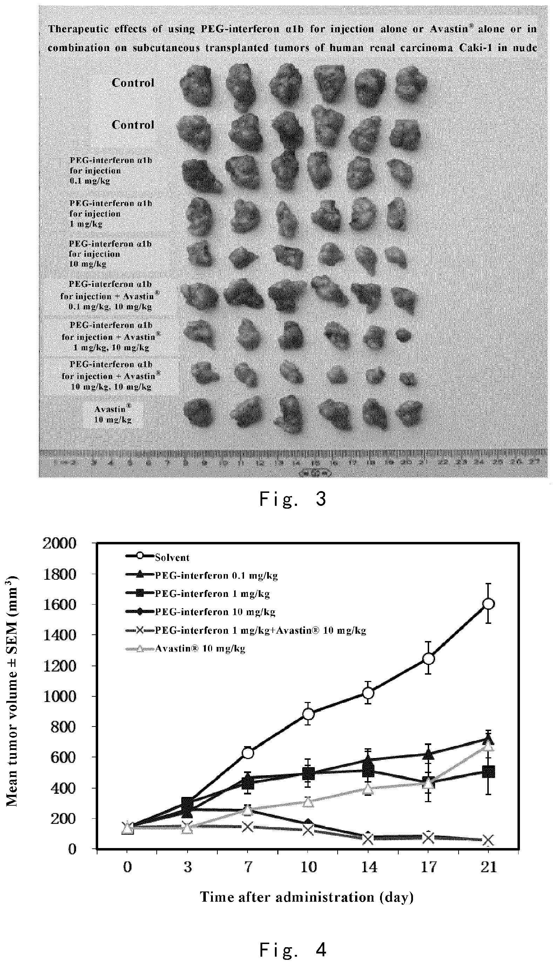

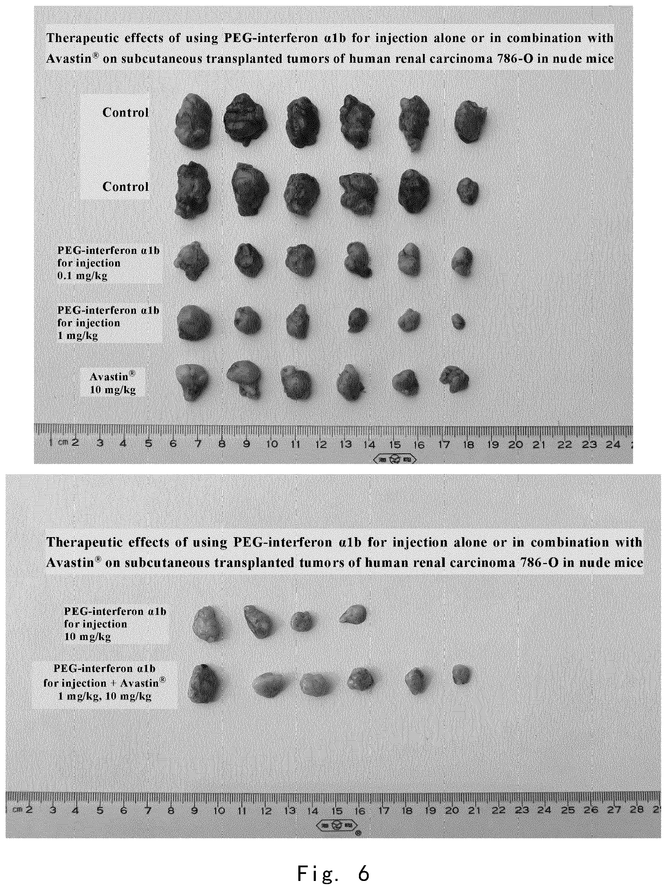

The results were shown in Table 2 and Figures 4, 5 and 6.

Table 2. Therapeutic effects of using PEGylated (PEG) interferon or Avastin alone or in combination on subcutaneous transplanted tumors of human renal carcinoma 786-O in nude mice.

| | | | Mean tumor volume (mm$^3$) | | Mean tumor % volume (mm$^3$) T/C | | | Tumor inhibition rate% | P value | Partial | Complete | Per group |
|---|---|---|---|---|---|---|---|---|---|---|---|---|
| Groups | Administration | Routes | DO | SEM | D21 | SEM | D21 | D21 | D21 | regression | regression | Number of animals |
| Solvent | D0,3,7,10,14,17 | SC | 141.5 ± | 2.6 | 1605.0 ± | 128.7 | - | - | - | 0 | 0 | 12 |
| PEGylated (PEG) interferon 0.1 mg/kg | D0,3,7,10,14,17 | SC | 144.3 ± | 2.9 | 721.8 ± | 54.5 | 39 | 61 | 0.000 | 0 | 0 | 6 |
| PEGylated (PEG) interferon 1 mg/kg | D0,3,7,10,14,17 | SC | 139.2 ± | 1.1 | 511.2 ± | 154.6 | 25 | 75 | *0.000 | 1 | 0 | 6 |
| PEGylated (PEG) interferon 10 mg/kg | D0,3,7,10,14,17 | SC | 141.5 ± | 4.1 | 55.7 ± | 18.2 | -61 | 161 | 0.000 | 4 | 2 | 6 |
| PEGylated (PEG) interferon 1 mg/kg+ Avastin 10 mg/kg | D0,3,7,10,14,17/ D0,3,7,10,14,17 | SC/IV | 143.3 ± | 2.7 | 61.4 ± | 13.8 | -57 | 157 | 0.000 | 5 | 1 | 6 |
| Avastin 10 mg/kg | D0,3,7,10,14,17 | IV | 137.6 ± | 4.4 | 675.6 ± | 81.6 | 37 | 63 | **0.000 | 0 | 0 | 6 |

D0: time of first administration; the first dose of Avastin was doubled; SC: subcutaneous injection; IV: intravenous injection; P value referred to the comparison with solvent; *P < 0.05, **P < 0.01, compared with the dose group PEGylated (PEG) 1 mg/kg + Avastin 10 mg/kg.

PEGylated (PEG) interferon (0.1, 1, 10 mg/kg, SC, twice a week, 6 times in total) dose-dependently inhibited the growth of subcutaneous transplanted tumors of human renal carcinoma 786-O in nude mice, and the tumor inhibition rates were 61%, 75% and 161%, respectively, with 1/6 of tumors regressed partially in the 1 mg/kg group, 4/6 of tumors regressed partially and 2/6 of tumors regressed completely in the 10 mg/kg group (D21), and by the end of the experiment (D42), 2/6 of tumors still regressed partially in the 10 mg/kg group. The tumor inhibition rate of Avastin® (10 mg/kg, IV, 2 times a week, 6 times in total) on 786-O subcutaneous transplanted tumors was 63% (D21).

When PEGylated (PEG) interferon (1 mg/kg, SC, 2 times a week, 6 times in total) was used in combination with Avastin® (10 mg/kg, IV, 2 times a week, 6 times in total), the tumor inhibition rate of 786-O was much significantly increased to 157% ($P < 0.05$, compared with monotherapy), with 5/6 of tumors regressed partially and 1/6 of tumors regressed completely (D21); and by the end of the experiment (D42), 1/6 of tumors still regressed partially. The results showed that PEGylated (PEG) interferon combined with Avastin had a very significant synergistic effect in the treatment of subcutaneous transplanted tumors of human renal carcinoma 786-O in nude mice, and even achieved the therapeutic effects of partial or complete regression.

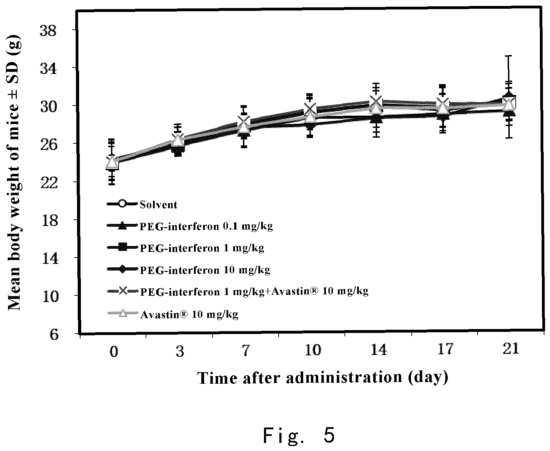

In addition, tumor-bearing mice showed good tolerance to the drugs when they were used in combination, and no symptoms such as weight loss occurred. Especially as shown in Figure 5, the body weights of the mice in the combination group of PEGylated (PEG) interferon $\alpha$1b (1 mg/kg, SC, 2 times a week, 6 times in total) and Avastin (10 mg/kg, IV, 2 times a week, 6 times in total) were higher than the body weights of the mice in the PEG-interferon alone group and the Avastin alone group, which suggested that when PEG-interferons and Avastin were used in combination, it could not only effectively improve the therapeutic effects, but also significantly reduce the corresponding side effects.

### 9. Conclusion

As shown in Figures 4, 5 and 6, PEGylated (PEG) interferon (0.1, 1, 10 mg/kg, SC, 2 times a week, 6 times in total) dose-dependently inhibited the growth of subcutaneous transplanted tumors of human renal carcinoma 786-O in nude mice, causing partial or complete regression of the tumor. Avastin® (10 mg/kg, IV, 2 times a week, 6 times in total) was also effective on 786-O subcutaneous transplanted tumors.

When used in combination, PEGylated (PEG) interferon (1 mg/kg, SC, 2 times a week, 6 times in total) significantly enhanced the therapeutic effects of Avastin® (10 mg/kg, IV, 2 times a week, 6 times in total) on 786-O subcutaneous transplanted tumors ($P < 0.05$, compared with monotherapy). Unexpectedly, the toxicity of the drugs not only did not increase significantly when they were used in combination, but also decreased significantly.

### Example 3

**Therapeutic effects of using PEGylated (PEG) interferon $\alpha$1b for injection alone or in combination with Avastin on subcutaneous transplanted tumors of human liver cancer Huh-7 in nude mice**

#### 1. Summary

The therapeutic effects of using PEGylated (PEG) interferon $\alpha$1b for injection (referred to as "PEGylated (PEG) interferon") alone or in combination with Avastin on subcutaneous transplanted tumors of human liver cancer Huh-7 in nude mice were evaluated. PEGylated (PEG) interferon (0.1, 1, 10 mg/kg, SC, 2 times a week, 5 times in total); Avastin® (10 mg/kg, IV, 2 times a week, 5 times in total); or PEGylated (PEG) interferon (1 mg/kg, SC, 2 times a week, 5 times in total) and Avastin® (10 mg/kg, IV, 2 times a week, 5 times in total) in combination were used.

#### 2. Experimental purpose

To evaluate the therapeutic effects of using PEGylated (PEG) interferon alone or in combination with Avastin on subcutaneous transplanted tumors of human liver cancer Huh-7 in nude mice.

#### 3. Test drugs

##### 3.1 Drug Information

PEGylated (PEG) interferon $\alpha$1b for injection (referred to as "PEGylated (PEG) interferon"): the same as that in Example 1.

Avastin (Bevacizumab injection): the same as that in Example 1.

### 3.2 Suppliers

The same as those in Example 1.

### 3.3 Drug formulation

Each bottle of PEGylated (PEG) interferon was added with 120 µl of sterile water for injection at a concentration of 1 mg/ml, prepared before use, and diluted with normal saline; Avastin was at a concentration of 25 mg/ml and was directly diluted with normal saline to the desired concentration.

### 4. Cells

Human liver cancer Huh-7 cells were purchased from the Chinese Academy of Sciences Cell Bank. Huh-7 cells were cultured in a 10-cm culture dish by adherent culture method, and the culture conditions were RPMI 1640 medium with 10% fetal bovine serum, penicillin and streptomycin, and cultured at 37°C in an incubator with air containing 5% $CO_2$. Cell passage was carried out 2-3 times a week, and when cells were in exponential growth phase, the cells were then digested with trypsin, collected, counted and seeded.

### 5. Experimental animals

[D000521]BALB/c-Foxnlnu/Nju mice, 6-7 weeks, ♀, were purchased from Jiangsu Jicui Yaokang Biotechnology Co., Ltd. Production license number: SCXK (Su) 2018-0008; animal certificate number 201900949. Breeding environment: SPF grade.

### 6. Experimental steps

Nude mice were subcutaneously inoculated with $6\times10^6$ human liver cancer Huh-7 cells. After the tumor grew to 100-150 mm$^3$, the animals were grouped according to tumor volume (D0). Mice were administered subcutaneously (SC) or intravenously (IV), twice a week (BIW); the administration volume was 10 mL/kg; the solvent group was administered with the same volume of "solvent" (normal saline); specific dosage and administration regimen were shown in Table 3. The tumor volumes were measured twice a week, the mice were weighed, and the data were recorded.

### 7. Statistical analysis

Two-tailed Student's t-test was used to compare tumor volumes between the two groups, and P<0.05 was defined as a statistically significant difference.

### 8. Results

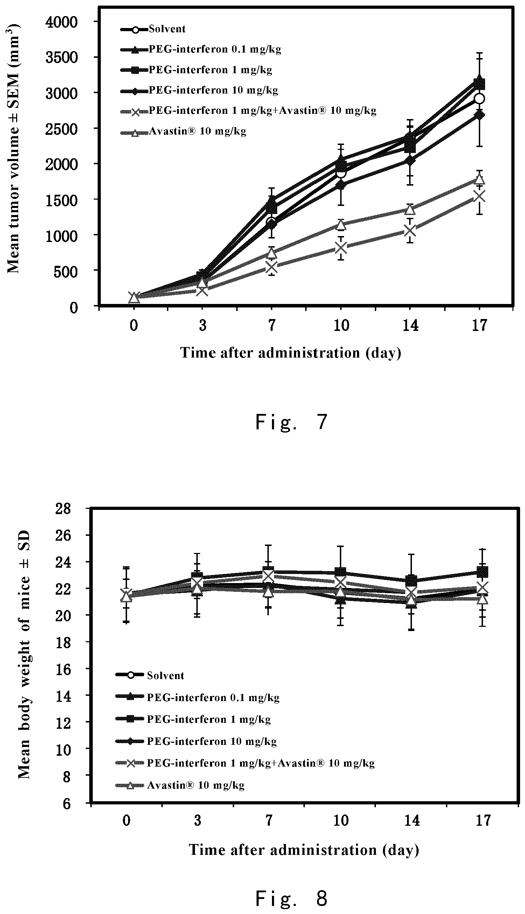

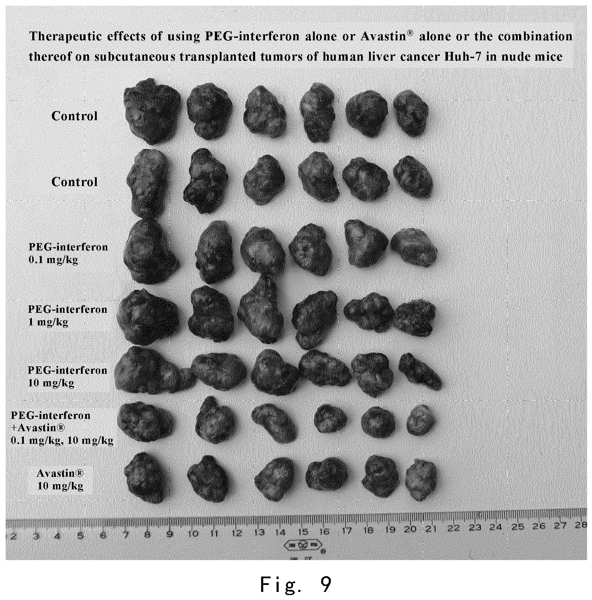

The results were shown in Table 3 and Figures 7, 8 and 9.

Table 3. Therapeutic effects of using PEGylated (PEG) interferon or Avastin alone or in combination on subcutaneous transplanted tumors of human liver cancer Huh-7 in nude mice.

| Groups | Administration | Routes | Mean tumor volume (mm$^3$) | | Mean tumor volume (mm$^3$) | | % T/C | Tumor inhibition rate% | P value | Per group |
|---|---|---|---|---|---|---|---|---|---|---|
| | | | D0 | SEM | D17 | SEM | D17 | D17 | D17 | Number of animals |
| Solvent | D0,3,7,10,14 | SC | 119.5 ± | 1.3 | 2916.2 ± | 164.1 | - | - | - | 12 |
| PEGylated (PEG) interferon 0.1 mg/kg | D0,3,7,10,14 | SC | 117.7 ± | 2.0 | 3188.0 ± | 282.5 | 110 | -10 | 0.383 | 6 |
| PEGylated (PEG) interferon 1 mg/kg | D0,3,7,10,14 | SC | 119.8 ± | 2.6 | 3110.6 ± | 438.0 | 107 | -7 | * 0.618 | 6 |
| PEGylated (PEG) interferon 10 mg/kg | D0,3,7,10,14 | SC | 123.8 ± | 1.9 | 2681.9 ± | 435.7 | 91 | 9 | 0.539 | 6 |
| PEGylated (PEG) interferon 1 mg/kg+ Avastin 10 mg/kg | D0,3,7,10,14/ D0,3,7,10,14 | SC/IV | 120.5 ± | 1.7 | 1549.1 ± | 260.9 | 51 | 49 | 0.000 | 6 |
| Avastin 10 mg/kg | D0,3,7,10,14 | IV | 122.7 ± | 1.8 | 1791.6 ± | 105.7 | 60 | 40 | 0.000 | 6 |

D0: time of first administration; the first dose of Avastin was doubled; SC: subcutaneous injection; IV: intravenous injection; P value referred to the comparison with solvent; *P < 0.05, compared with the dose group PEGylated (PEG) 1 mg/kg + Avastin 10 mg/kg.

PEGylated (PEG) interferon (0.1, 1, 10 mg/kg, SC, twice a week, 5 times in total) dose-dependently inhibited the growth of subcutaneous transplanted tumors of human liver cancer Huh-7 in nude mice, and the tumor inhibition rates were -10%, -7% and 9%, respectively. The tumor inhibition rate of Avastin® (10 mg/kg, IV, 2 times a week, 5 times in total) on Huh-7 subcutaneous transplanted tumors was 40%.

When PEGylated (PEG) interferon (1 mg/kg, SC, 2 times a week, 5 times in total) was used in combination with Avastin® (10 mg/kg, IV, 2 times a week, 5 times in total), the tumor inhibition rate of Huh-7 was increased to 49%. PEGylated (PEG) interferon had certain synergistic effect on subcutaneous transplanted tumors of human liver cancer Huh-7 in nude mice treated with Avastin.

In addition, tumor-bearing mice showed good tolerance to the drugs when they were used in combination, and no symptoms such as weight loss occurred. As shown in Figure 8, the body weights of the mice in the combination group of PEGylated (PEG) interferon $\alpha$1b (1 mg/kg, SC, 2 times a week, 6 times in total) and Avastin (10 mg/kg, IV, 2 times a week, 6 times in total) were basically indistinguishable from the body weights of the mice in the PEG-interferon (1 mg/kg) alone group and the Avastin (10 mg/kg) alone group. Moreover, the body weights of the combination group were higher than those of Avastin (10 mg/kg) alone group, which suggested that the side effects of Avastin administration could be further reduced when it was used in combination.

### 9. Conclusion

As shown in Figures 7, 8 and 9, PEGylated (PEG) interferon (0.1, 1, 10 mg/kg, SC, 2 times a week, 5 times in total) had no significant inhibitory effect on the growth of subcutaneous transplanted tumors of human liver cancer Huh-7 in nude mice. Avastin® (10 mg/kg, IV, 2 times a week, 5 times in total) was effective on Huh-7 subcutaneous transplanted tumors.

When used in combination, PEGylated (PEG) interferon had a synergistic effect on subcutaneous transplanted tumors of human liver cancer Huh-7 in nude mice treated with Avastin. In addition, tumor-bearing mice showed good tolerance to the drugs when used in combination without increased toxicity.

### Discussion

Interferon is a kind of highly active protein molecules with broad-spectrum antiviral proliferation activity, antitumor activity, immunomodulatory activity and so on. In the field of tumor therapy, ordinary interferons have been approved by the US FDA and European EMA for the treatment of hairy cell leukemia, chronic myeloid leukemia, renal cancer, Kaposi's sarcoma, melanoma, follicular lymphoma, multiple myeloma, cervical intraepithelial neoplasia (Elena Garcia-Martinez, etc. Trial Watch: Immunostimulation with recombinant cytokines for cancer therapy, ONCOIMMUNOLOGY, 2018, VOL. 7, NO. 6: e1433982, 16 pages; Xiang Chen et al., Clinical research progress of immunotherapy for renal cancer, CHINESE JOURNAL OF UROLOGY, 2017 VOL. 38 NO. 9: 717-720). Besides, Interferon they also has certain curative effects in the treatment of lung cancer, digestive tract cancer, prostate cancer, head and neck squamous cell carcinoma and other tumors.

Although ordinary interferons are safe and effective preparations, there are still some problems, especially serious side effects. The ordinary interferons currently used in clinical practice are easy to be filtered by the glomerulus due to their small molecular weights (below 20 kDa), resulting in a half-life of only 1.5-2 hours in the human body. In order to achieve the desired therapeutic effect, a general clinical treatment of ordinary interferons uses high doses and multiple injections, which leads to poor patient compliance and is prone to side effects.

Taking melanoma as an example, it is necessary to subcutaneously inject ordinary interferons 20 million IU/m$^2$ (approximately equal to 400 $\mu$g/person) for 5 days/week, then 10 million IU/m$^2$ (approximately 200 $\mu$g/person) after 1 month, and maintain the treatment 3 times a week for 48 weeks. The treatment can prolong the survival of patients, but the adverse reactions of administering high-dose ordinary interferons are very obvious. At least 50% of patients need to delay or reduce the dose, so it can only be used for high-risk patients.

The adverse reactions of ordinary interferon $\alpha$ in the human body are systemic (Xue-lin, Cao, Mechanism of interferon adverse reactions, BIOMEDICAL ENGINEERING AND CLINICAL MEDICINE, 2010, VOL. 14, NO. 4: 340-342): in the early stage of therapy, most patients will appear influenza-like syndrome, and clinical symptoms include fever, chills, headache, myalgia, gastrointestinal pain, etc. Nausea, vomiting and loss of appetite are common gastrointestinal reactions in the early stage of ordinary interferon $\alpha$ therapy. Fatigue occurs in more than 70% of patients during ordinary interferon $\alpha$ therapy. The main adverse reactions of the neuropsychiatric system are mental disorders, depression, trance, anxiety, irritability, etc. About 20% of patients receiving ordinary interferon $\alpha$ therapy may experience mild to moderate decreases in the total number of white blood cells, neutrophils, and platelets. Skin rash is the most common mucocutaneous lesion caused by ordinary interferon $\alpha$, which is often manifested as a non-specific rash with pruritus; other manifestations include erythema at the injection site, skin ulceration, oral mucosal ulceration, cheilitis, and etc. Ordinary interferon $\alpha$ therapy can lead to thyroid dysfunction through autoimmune and non-autoimmune mechanisms, and hypothyroidism is the most common thyroid dysfunction. Ordinary interferon $\alpha$ can also induce autoimmune damage to islet $\beta$ cells, thereby inducing diabetes. Other less common adverse reactions are interstitial pneumonia, retinopathy, hearing impairment and tinnitus, hair loss, diarrhea, and weight loss.

In order to improve the therapeutic effects of ordinary interferon $\alpha$ in the treatment of tumors and reduce the side effects, a beneficial exploration of the combination of ordinary interferon $\alpha$ with other tumor drugs has been carried out (Jun Guo, Advances in medical therapy of metastatic renal cancer, THE SECOND CHINESE CONGRESS OF MEDICAL ONCOLOGY, 2008: 33-36). Among them, the regimen of using Bevacizumab combined with ordinary interferon $\alpha$ in the treatment of advanced renal cancer is the most successful. Two independent clinical trials have confirmed that using Bevacizumab combined with ordinary interferon $\alpha$ in the treatment of renal cancer can significantly prolong the PFS of patients compared with using ordinary interferons alone (10.2 vs 5.4 months, 8.5 vs 5.2 months). Based on the above results, in 2009, the US FDA approved Bevacizumab combined with ordinary interferons for the treatment of advanced renal cancer. However, the highest incidence of adverse reactions at grade 3 and above can reach 80% (Brian I. Rini, etc. Phase III Trial of Bevacizumab Plus Interferon Alfa Versus Interferon Alfa Monotherapy in Patients With Metastatic Renal Cell Carcinoma: Final Results of CALGB 90206, JOURNAL OF CLINICAL ONCOLOGY, 2010, VOL28, No. 13: 2137-2143), so patients' compliance is poor, which limits the application of the regimen.

Human interferon $\alpha$1b is a protein composed of 166 amino acids produced by human leukocytes, and its relative molecular weight is 19.382 kDa. Studies have shown that $\alpha$1b type interferon is the main type of interferons produced by white blood cells of Chinese people after being attacked by a virus.

In the early 1980s, Yunde Hou et al. from the Institute of Virology of the Academy of Medical Science cloned the $\alpha$1b interferon gene for the first time. This first genetically engineered protein drug in China, recombinant human interferon $\alpha$1b, was jointly developed by the Institute of Virology and Shanghai Institute of Biological Products, and is one of the few new drugs with independent intellectual property rights in China.

Compared with ordinary interferon $\alpha_2$ products, ordinary interferon $\alpha_1$ has relatively low biological activity *in vitro*, but has a stronger *in vivo* effect with a wide range and less side effects. For example, the specific activities of ordinary interferon $\alpha$2a and 2b *in vitro* are both $1 \times 10^8$ IU/mg, while the specific activity of ordinary interferon $\alpha_{1b}$ is only $1 \times 10^7$ IU/mg, which is 10% of the former two. In clinical application, the same weight of ordinary interferon $\alpha_{1b}$ has the same therapeutic effects of ordinary interferon $\alpha$2a or 2b, and the side effects of ordinary interferon $\alpha_{1b}$ are significantly less than the latter two (Kui-tang Tong, Interferon $\alpha$ treatment of melanoma, THE 17th NATIONAL ACADEMIC CONFERENCE ON INTERFERONS AND CYTOKINES, 2011:2-6). In the clinical trial of ordinary interferon $\alpha_{1b}$ in the treatment of hairy cell leukemia, the effective rate was 63.64%, the remission rate was 36.36%, and the adverse reaction was only a short-term low-grade fever, which subsided without medication, and other adverse reactions were not obvious. All adverse reactions were tolerated, and no patient discontinued its medication.

The results of phase I and II clinical trials in the United States showed that high-dose (500 $\mu$g/day) injection of ordinary interferon $\alpha$1b had a certain therapeutic effect in the treatment of patients with metastatic renal cancer and multiple myeloma, and the adverse reactions were milder compared with other ordinary interferons (P. Masci, etc. Gene Modulatory Effects, Pharmacokinetics, and Clinical Tolerance of Interferon $\alpha$-1b: A Second Member of the Interferon $\alpha$ Family. Clinical Pharmacology & Therapeutics. 2007. Vol.81, No.3:354-361).

Ordinary interferon $\alpha$1b, like other ordinary interferons, also has the defects of being easily filtered by the glomerulus and having a short half-life in the human body due to its small molecular weight. In order to prolong the half-life and improve its therapeutic effects, ordinary interferon $\alpha$1b was modified with the second-generation polyethylene glycol modifier to obtain an interferon $\alpha$1b modified with a single PEG, which was named as PEGylated (PEG) interferon $\alpha$1b. Studies have shown that its molecular weight is increased, and its half-life is significantly longer than that of unmodified ordinary interferon. The half-life of PEGylated (PEG) interferon $\alpha$1b in rats is 13.98 hours, while that of ordinary interferon $\alpha$1b is 1.84 hours; the half-life of PEGylated (PEG) interferon $\alpha$1b in cynomolgus monkeys is 22.03 $\pm$ 3.32 hours, while that of ordinary interferon $\alpha$1b is 3.27 $\pm$ 1.48 h. According to relevant research reports on PEG-modified recombinant therapeutic protein drugs on the market, the increase in molecular weight of such drugs is positively correlated with the improvement of their clinical efficacy.

In the present invention, preclinical pharmacodynamic studies *in vitro* and *in vivo* were conducted by using PEGylated (PEG) interferon $\alpha$1b alone or using it in combination with other drugs.

The research results showed that the combined use of PEGylated (PEG) interferon $\alpha$1b with Avastin, and other anti-tumor drugs not only had a synergistic effect in terms of efficacy, but also significantly reduced various side effects synergistically.

In the renal carcinoma caki-1 tumor model *in vivo* of nude mice, the monotherapy of PEGylated (PEG) interferon $\alpha$1b in any group of low, medium and high doses had therapeutic effects on renal cancer, and the therapeutic effect were obviously dose-dependent. In addition, a significant reduction in toxic and side effects was observed.

While in the combination regimen with monoclonal antibodies, the combined use of low, medium or high doses of PEGylated (PEG) interferon $\alpha$1b with Avastin had synergistic inhibitory effects on renal carcinoma Caki-1, wherein the combination group of PEGylated (PEG) interferon $\alpha$1b in medium-dose (1 mg/kg) and Avastin had a significant synergistic effect (69% inhibition rate in the combination group vs 46% inhibition rate in the PEGylated (PEG) interferon α1b alone group vs 42% inhibition rate in the Avastin alone group).

However, in the liver cancer Huh-7 model of nude mice, the PEGylated (PEG) interferon α1b alone group did not show obvious inhibitory effects on tumors, thus it can be seen that although PEGylated (PEG) interferon α1b has anti-tumor activity, there are still some or great differences in the therapeutic effects of different tumors. However, when the PEG-interferon was used in combination with Avastin, it showed a certain synergistic effect.

Although PEGylated (PEG) interferon α2a and α2b have obvious advantages over ordinary interferons in the therapeutic effects of chronic hepatitis B and C, and have achieved great success. But in the field of cancer treatment, the effects are quite different.

In a phase III clinical study (T. K. Eigentler, etc. Adjuvant treatment with pegylated interferon α-2a versus low-dose interferon α-2a in patients with high risk melanoma: a randomized phase III DeCOG trial, Annals of Oncology, 2016, 27:1625-1632), the efficacy and safety of administering PEGylated (PEG) interferon α2a or ordinary interferon α2a to high-risk melanoma patients were compared, and found that PEGylated (PEG) interferon α2a was not more effective than ordinary interferon α2a, while the side effects were more severe.

In another phase III clinical study (THOMAS M. H, etc. U.S. Food and Drug Administration Approval: Peginterferon-alfa-2b for the Adjuvant Treatment of Patients with Melanoma, The oncologist, 2012;17:1323-1328), the mean recurrence-free survival of melanoma patients treated with PEGylated (PEG) interferon α2b in adjuvant therapy was 34.8 months, which was significantly longer than the 25.5 months of the observation group, but there was no significant improvement in overall survival. Although the incidence of serious adverse reactions in the treatment group was almost twice that of the observation group (33% vs 15%), in 2011 the US FDA still approved PEGylated (PEG) interferon α2b for adjuvant therapy of malignant melanoma patients with regional lymph nodes involvement. The PEGylated (PEG) interferon α2b was administered subcutaneously at 6 μg/kg every week, and maintained at 3 μg/kg every week after 8 consecutive injections of 6 μg/kg (NCCN Clinical Practice Guidelines in Oncology - Malignant Melanoma, 2nd Edition, 2018: MS-20). At present, there is only one approved regimen of PEGylated (PEG) interferon drugs for tumor indications, which shows that in the field of tumor treatment, the effects of different PEGylated (PEG) interferon subtypes and different tumor types on the therapeutic effects are significantly different, thus the results cannot be simply obtained by analogy.

Unexpectedly, unlike PEGylated (PEG) interferon α2a, the study of the present invention shows that the combined use of a PEGylated (PEG) interferon α1b with Avastin can inhibit tumors synergistically, and can significantly reduce the incidence or extent of their respective side effects (e.g. weight loss, fever, gastrointestinal adverse reactions, rash, etc.) at the same time.

All documents mentioned in the present invention are incorporated by reference herein as if each document were incorporated separately by reference. Furthermore, it should be understood that after reading the foregoing teachings of the invention, various changes or modifications may be made to the invention by those skilled in the art and that these equivalents also fall in the scope of the claims appended to this application.

**Claims**

1. Use of a combination of active ingredients in preparation of a pharmaceutical composition or kit for synergistic treatment of renal carcinoma, wherein the combination of active ingredients comprises a first active ingredient of PEG-interferon (or PEGylated interferon) and a second active ingredient of a proto-oncogene product targeting inhibitor.

2. The use of claim 1, wherein the PEG-interferon is a PEG-interferon α1b.

3. The use of claim 1, wherein a mass ratio of the PEG-interferon to the proto-oncogene product targeting inhibitor is 1:100 to 500:1, preferably 1:20 to 200:1, and more preferably 1:10 to 100:1 or 1:5 to 50:1;
   or, a ratio (IU/mg) of the PEG-interferon to the proto-oncogene product targeting inhibitor is 1,000 IU/mg to 100 million IU/mg, preferably 10,000 IU/mg to 10 million IU/mg, and more preferably 100,000 IU/mg to 5 million IU/mg or 200,000 IU/mg to 3 million IU/mg.

4. The use of claim 1, wherein the proto-oncogene product targeting inhibitor is selected from the group consisting of: an EGFR inhibitor, and combinations thereof.

5. The use of claim 1, wherein the proto-oncogene product targeting inhibitor comprises Avastin.

6. A pharmaceutical composition, which comprises a PEG-interferon, a proto-oncogene product targeting inhibitor and a pharmaceutically acceptable carrier.

7. The pharmaceutical composition of claim 6, wherein the pharmaceutical composition further comprises an additional therapeutic drug (e.g. an antitumor agent).

8. A combination of active ingredients, which consists of a PEG-interferon and a proto-oncogene product targeting inhibitor.

9. A kit comprising:

   (a) a first preparation comprising a PEG-interferon and a pharmaceutically acceptable carrier;
   (b) a second preparation comprising a proto-oncogene product targeting inhibitor and a pharmaceutically acceptable carrier;
   (c) an instruction describing a method of combining the PEG-interferon with the proto-oncogene product targeting inhibitor for tumor treatment.

10. A non-therapeutic *in vitro* method for synergistic inhibition on growth of tumor cells, which comprises the following steps: adding the combination of active ingredients of claim 8 to a culture system for tumor cells, thereby synergistically inhibiting the growth of tumor cells.

Fig. 1

Fig. 2

Therapeutic effects of using PEG-interferon α1b for injection alone or Avastin® alone or in combination on subcutaneous transplanted tumors of human renal carcinoma Caki-1 in nude

Fig. 3

Fig. 4

Fig. 5

Therapeutic effects of using PEG-interferon α1b for injection alone or in combination with Avastin® on subcutaneous transplanted tumors of human renal carcinoma 786-O in nude mice

Fig. 6

Fig. 7

Fig. 8

Fig. 9

<div align="center">

**INTERNATIONAL SEARCH REPORT**

</div>

| | |
|---|---|
| | International application No. |
| | **PCT/CN2019/130747** |

| | |
|---|---|
| **A.** | **CLASSIFICATION OF SUBJECT MATTER** |
| | A61K 38/21(2006.01)i; A61K 39/00(2006.01)i; C07K 17/02(2006.01)i; A61P 35/00(2006.01)i |
| | According to International Patent Classification (IPC) or to both national classification and IPC |

| | |
|---|---|
| **B.** | **FIELDS SEARCHED** |
| Minimum documentation searched (classification system followed by classification symbols) | |
| A61K; C07K; A61P | |
| Documentation searched other than minimum documentation to the extent that such documents are included in the fields searched | |
| Electronic data base consulted during the international search (name of data base and, where practicable, search terms used) | |
| CNMED; TWABS; MOABS; TWMED; HKABS; CNABS; DWPI; CNKI; ISI Web of science: 培干扰素, 安维汀, 原癌基因产物靶向抑制剂, 干扰素, 聚乙二醇, 肾癌, EGFR抑制剂, 贝伐珠单抗, 抗肿瘤剂, bevacizumab, avastin, PEG, interferon, antitumor, renal carcinoma | |

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| Y | 史久华 (SHI, Jiuhua). "贝伐单抗加干扰素α治疗转移性肾细胞癌 (Non-official translation: Treatment of Metastatic Renal Cell Carcinoma Combining Bevacizumab and Interferon alpha)" 国际生物制品学杂志 (*International Journal of Biologicals*), Vol. 32, No. 6, 31 December 2009 (2009-12-31), ISSN: 1673-4211, p. 305, last paragraph | 1-10 |
| Y | CN 1634993 A (SHANGHAI INSTITUTE OF BIOLOGICAL PRODUCTS) 06 July 2005 (2005-07-06) abstract, and claims 1-3 | 1-10 |
| Y | 杨宇峰 等 (YANG, Yufeng et al.). "聚乙二醇修饰重组人干扰素α1b的制备及其性质 (Preparation and Properties of PEGylated Recombinant Human Interferon α1b)" 中国生物制品学杂志 (*Chinese Journal of Biologicals*), Vol. 23, No. 6, 30 June 2010 (2010-06-30), ISSN: 1004-5503, abstract, page 615, left-hand column, paragraph 1 to page 618, left-hand column, paragraph 1 | 1-10 |

| | | |
|---|---|---|
| ☑ Further documents are listed in the continuation of Box C. | ☑ See patent family annex. | |

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **02 June 2020** | **23 June 2020** |

| Name and mailing address of the ISA/CN | Authorized officer |
|---|---|
| **China National Intellectual Property Administration (ISA/CN)** **No. 6, Xitucheng Road, Jimenqiao Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2019/130747** |

**C.    DOCUMENTS CONSIDERED TO BE RELEVANT**

| Category* | Citation of document, with indication, where appropriate, of the relevant passages | Relevant to claim No. |
| --- | --- | --- |
| A | CN 105007896 A (ABBOTT CARDIOVASCULAR SYSTEMS INC.) 28 October 2015 (2015-10-28) <br> abstract, claims 1-30 | 1-10 |
| A | EP 2789628 A2 (ANGIOCHEM INC.) 15 October 2014 (2014-10-15) <br> abstract, and claims 1-15 | 1-10 |
| A | CN 105246915 A (ALDER BIOPHARMACEUTICALS, INC.) 13 January 2016 (2016-01-13) <br> abstract, and claims 1-23 | 1-10 |

Form PCT/ISA/210 (second sheet) (January 2015)

EP 4 043 027 A1

**INTERNATIONAL SEARCH REPORT**
Information on patent family members

International application No.

**PCT/CN2019/130747**

| Patent document cited in search report | | | Publication date (day/month/year) | Patent family member(s) | | | Publication date (day/month/year) |
|---|---|---|---|---|---|---|---|
| CN | 1634993 | A | 06 July 2005 | CN | 1289528 | C | 13 December 2006 |
| CN | 105007896 | A | 28 October 2015 | US | 9452138 | B2 | 27 September 2016 |
| | | | | WO | 2014106116 | A8 | 18 June 2015 |
| | | | | JP | 2016504369 | A | 12 February 2016 |
| | | | | US | 2014186446 | A1 | 03 July 2014 |
| | | | | CN | 105007896 | B | 09 April 2019 |
| | | | | EP | 2938323 | A2 | 04 November 2015 |
| | | | | WO | 2014106116 | A3 | 31 July 2014 |
| | | | | JP | 6533932 | B2 | 26 June 2019 |
| | | | | US | 2017042816 | A1 | 16 February 2017 |
| | | | | WO | 2014106116 | A2 | 03 July 2014 |
| EP | 2789628 | A2 | 15 October 2014 | EP | 2164866 | B1 | 14 May 2014 |
| | | | | WO | 2008144919 | A1 | 04 December 2008 |
| | | | | AU | 2008255556 | A1 | 04 December 2008 |
| | | | | EP | 2789628 | A3 | 04 March 2015 |
| | | | | EP | 2789628 | A2 | 15 October 2014 |
| | | | | EP | 2164866 | A4 | 09 June 2010 |
| | | | | CA | 2688344 | C | 03 September 2019 |
| | | | | PL | 2164866 | T3 | 31 October 2014 |
| | | | | AU | 2008255556 | B2 | 07 August 2014 |
| | | | | EP | 2164866 | A1 | 24 March 2010 |
| | | | | CN | 101815724 | B | 25 February 2015 |
| | | | | CA | 2688344 | A1 | 04 December 2008 |
| | | | | CN | 101815724 | A | 25 August 2010 |
| | | | | JP | 2016006089 | A | 14 January 2016 |
| | | | | US | 9365634 | B2 | 14 June 2016 |
| | | | | JP | 5844971 | B2 | 20 January 2016 |
| | | | | US | 2010297120 | A1 | 25 November 2010 |
| | | | | US | 2009016959 | A1 | 15 January 2009 |
| | | | | JP | 2010528058 | A | 19 August 2010 |
| | | | | ES | 2789628 | T3 | 28 August 2014 |
| | | | | AU | 2014259519 | A1 | 27 November 2014 |
| | | | | IN | 200907637 | P4 | 16 July 2010 |
| CN | 105246915 | A | 13 January 2016 | KR | 20150140685 | A | 16 December 2015 |
| | | | | WO | 2014153117 | A3 | 08 January 2015 |
| | | | | TW | 201438737 | A | 16 October 2014 |
| | | | | WO | 2014153117 | A2 | 25 September 2014 |
| | | | | EP | 2964673 | A2 | 13 January 2016 |
| | | | | JP | 2016516052 | A | 02 June 2016 |
| | | | | WO | 2014153166 | A3 | 04 December 2014 |
| | | | | WO | 2014153166 | A2 | 25 September 2014 |
| | | | | CA | 2904743 | A1 | 25 September 2014 |
| | | | | EP | 2964673 | A4 | 22 February 2017 |
| | | | | TW | 201444868 | A | 01 December 2014 |
| | | | | WO | 2014153117 | A9 | 27 November 2014 |
| | | | | US | 2014271459 | A1 | 18 September 2014 |
| | | | | AR | 095556 | A1 | 28 October 2015 |

Form PCT/ISA/210 (patent family annex) (January 2015)

**EP 4 043 027 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 200410067652

**Non-patent literature cited in the description**

- **ELENA GARCIA-MARTINEZ.** Trial Watch: Immunostimulation with recombinant cytokines for cancer therapy. *ONCOIMMUNOLOGY,* 2018, vol. 7 (6), e1433982, , 16
- **XIANG CHEN et al.** Clinical research progress of immunotherapy for renal cancer. *CHINESE JOURNAL OF UROLOGY,* 2017, vol. 38 (9), 717-720
- **XUE-LIN ; CAO.** Mechanism of interferon adverse reactions. *BIOMEDICAL ENGINEERING AND CLINICAL MEDICINE,* 2010, vol. 14 (4), 340-342
- **JUN GUO.** Advances in medical therapy of metastatic renal cancer. *THE SECOND CHINESE CONGRESS OF MEDICAL ONCOLOGY,* 2008, 33-36
- **BRIAN I. RINI.** Phase III Trial of Bevacizumab Plus Interferon Alfa Versus Interferon Alfa Monotherapy in Patients With Metastatic Renal Cell Carcinoma: Final Results of CALGB 90206. *JOURNAL OF CLINICAL ONCOLOGY,* 2010, vol. 28 (13), 2137-2143
- **KUI-TANG TONG.** Interferon $\alpha$ treatment of melanoma. *THE 17th NATIONAL ACADEMIC CONFERENCE ON INTERFERONS AND CYTOKINES,* 2011, 2-6
- **P. MASCI.** Gene Modulatory Effects, Pharmacokinetics, and Clinical Tolerance of Interferon $\alpha$-1b: A Second Member of the Interferon $\alpha$ Family. *Clinical Pharmacology & Therapeutics,* 2007, vol. 81 (3), 354-361
- **T. K. EIGENTLER.** Adjuvant treatment with pegylated interferon $\alpha$-2a versus low-dose interferon $\alpha$-2a in patients with high risk melanoma: a randomized phase III DeCOG trial. *Annals of Oncology,* 2016, vol. 27, 1625-1632
- **THOMAS M. H.** U.S. Food and Drug Administration Approval: Peginterferon-alfa-2b for the Adjuvant Treatment of Patients with Melanoma. *The oncologist,* 2012, vol. 17, 1323-1328
- NCCN Clinical Practice Guidelines in Oncology - Malignant Melanoma. 2018